# EUROPEAN PATENT APPLICATION

(11) **EP 1 967 161 A1**
(43) Date of publication of application: **10.09.2008**
(21) Application number: 08102152.9
(22) Date of filing: 29.02.2008
(51) Int. Cl.: A61F 2/16, A61M 5/315

(54) **Lens delivery system**

(30) Priority: 06.03.2007 US 682622
(71) Applicant: ALCON RESEARCH, LTD., 76134-2099 TX, Texas Fort Worth (US)
(72) Inventor: Downer, David A., Fort Worth, TX Texas 76137 (US)
(74) Representative: Hanna, Peter William Derek

(57) **Abstract**

An intraocular lens delivery system is provided having a plunger (16), an injector body (14) and a nozzle portion (12) connected to the injector body. A sliding gate (28) is attached to a flange (18) located on the proximal end of the injector body. The gate engages a receptor notch (34) in the plunger and prevents movement of the plunger until released.

## Description

This invention relates to intraocular lenses (IOLs) and more particularly to devices use to inject IOLs into an eye.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.

While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. The most commonly used injector cartridge design is illustrated in U.S. Patent No. 4,681,102 (Bartell), and includes a split, longitudinally hinged cartridge. Similar designs are illustrated in U.S. Patent Nos. 5,494,484 and 5,499,987 (Feingold) and 5,616,148 and 5,620,450 (Eagles, et al.). In an attempt to avoid the claims of U.S. Patent No. 4,681,102, several solid cartridges have been investigated, see for example U.S. Patent No. 5,275,604 (Rheinish, et al.) and 5,653,715 (Reich, et al.).

These devices all require that the lens be shipped separately from the cartridge. This requires that the lens be removed from its shipping container and placed in the cartridge prior to use. This requires additional handling of the lens, with the resulting potential for damage to the lens. Two prior art devices, disclosed in U.S. Patent No. 6,471,708 B1 (Green) and 7,156,854 (Brown, et al.) disclose lens delivery systems that is also suitable for use as a lens shipment container as well as a delivery device. As recognized in U.S. Patent Number 7,156,854, during shipment, the plunger can be inadvertently moved forward, which can adversely affect the lens. Accordingly, a need continues to exist for a combination lens shipment device and delivery system.

### Brief Summary of the Invention

The present invention improves upon prior art by providing a combination lens packaging/shipment container and delivery system having a plunger, an injector body and a nozzle portion connected to the injector body, in accordance with claims which follow. A sliding gate is attached to a flange located on the proximal end of the injector body. The gate engages a receptor slot in the plunger and prevents movement of the plunger until released.

It is accordingly an object of the present invention to provide a lens delivery system suitable for the storage, shipment and delivery of a lens into an eye without the use of any additional devices.

It is a further object of the present invention to provide a lens delivery system that is suitable for folding lenses made from a soft acrylic material.

It is a further object of the present invention to provide a lens delivery system having a slidable gate that limits movement of the plunger during storage and shipment.

Other objects, features and advantages of the present invention will become apparent with reference to the drawings, and the following description of the drawings and claims.

### Brief Description of the Drawings

FIG. 1 is an enlarged perspective view of a first embodiment of the lens delivery system of the present invention.
FIG. 2 is an enlarged partial perspective view of the proximal end of a first embodiment of the lens delivery system of the present invention looking distally.
FIG. 3 is an enlarged partial side elevational view of the proximal end of a first embodiment of the lens delivery system of the present invention.
FIG. 4 is an enlarged partial perspective view of the proximal end of the lens delivery system of the present invention looking proximally.
FIG. 5 is an enlarged top plan view of a gate that may be used with the lens delivery system of the present invention.
FIG. 6 is an enlarged end elevational view of a first embodiment of a gate that may be used with the lens delivery system of the present invention.
FIG. 7 is an enlarged partial perspective view of the proximal end of a second embodiment of the lens delivery system of the present invention looking distally.

### Detailed Description of the Preferred Embodiments

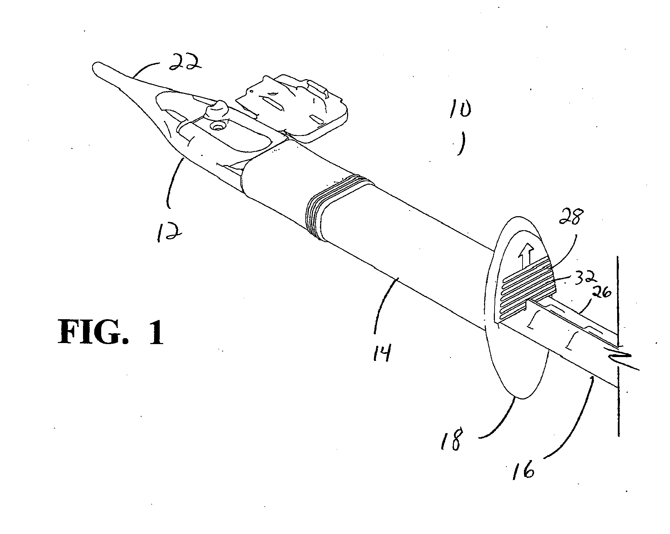

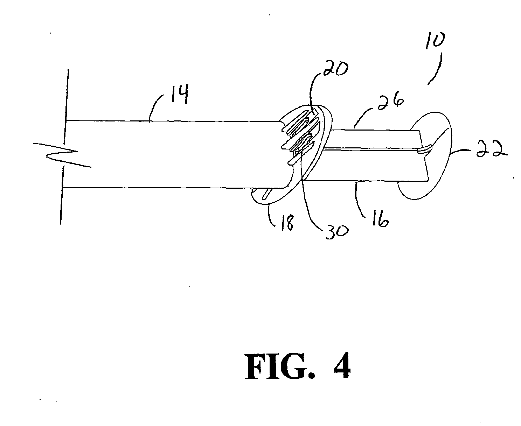

As best seen in FIGS. 1-3, lens delivery system 10 of the present invention generally includes nozzle portion 12, injector body 14 and plunger 16. Nozzle portion 12, body 14 and plunger 16 can be molded from any suitable thermoplastic, such as polypropylene, and the thermoplastic may contain a lubricity enhancing agent such as those disclosed in U.S. Pat. No. 5,716,364. Alternatively, nozzle portion 12 may be integrally formed with body 14. Nozzle portion 12 contains distal nozzle 22 that is preferably is round, oval or elliptical in cross-section and has a cross-sectional area of between around 1.0 mm2 to around 2.6 mm2 • Injector body 14 contains at least one finger flange 18. As best seen in FIG. 4, finger flange 18 contains at least one slot 20 that has the function described below. Plunger 16 generally consists of plunger rod 26 connected to proximally located plunger cap 24 that cooperates with finger flange 18 to provide a means to reciprocate plunger 16 within body 14 in a manner similar to the operation of a syringe.

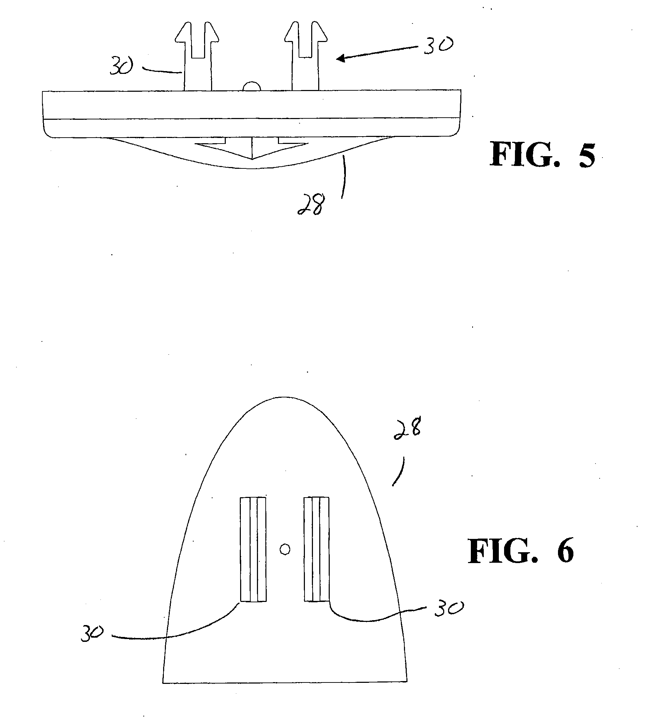

As best seen in FIGS. 5 and 6, gate 28 is of similar size and shape as finger flange 18 and contains at least one snap-locking tab 30 that are sized and shaped to fit within slot(s) 20 of finger flange 18, but tabs 30 are slightly shorter than slots 20. Gate 28 may also contain knurling 32 or other texturing opposite tabs 30 to provide the user with a more positive grip.

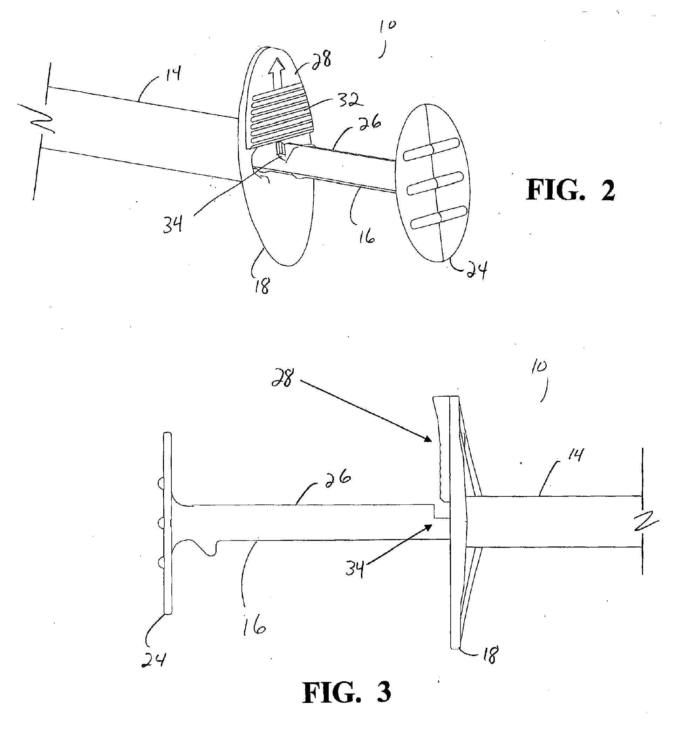

In use, tabs 30 on gate 28 are snapped into place within slots 20 on finger flange 18 so as to hold gate 28 against finger flange 18. Tabs 30, being shorter than slot(s) 20, allow gate 28 to slide upon finger flange 18 within slot(s) 20. Plunger 16 is inserted into body 14 so that receptor notch 34 on plunger rod 26 is aligned with gate 28, as seen in FIG. 3. Gate 28 is pushed downward toward plunger rod 26 until gate 28 engages receptor notch 34, as seen in FIG. 1. Once engaged with receptor notch 34, gate 28 prevent further movement of plunger rod 26 and plunger 16. To release plunger 16, gate 28 is pushed in the opposite direction until gate 28 is once again clear of receptor notch 34.

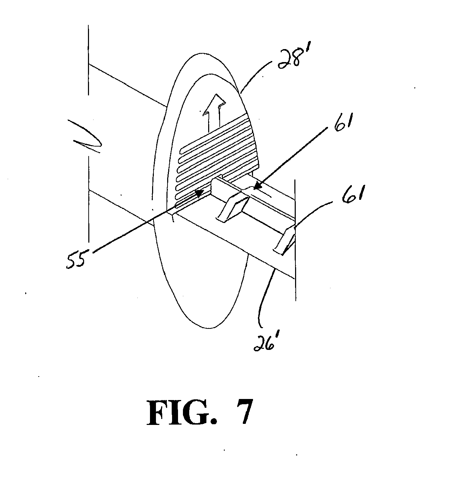

Alternatively, as seen in FIG. 7, gate 28' may contain notch 55 sized and shaped to fit over web 59 on plunger rod 26'. A rib 61 on plunger rod 26' engages gate 28' and prevents movement of plunger rod 26'. A series of ribs 61 can be placed at any desired location along plunger rod 26'.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An intraocular lens delivery system, comprising:
a) an injector body (14) having a hollow interior, the injector body further having a nozzle portion (12) having a hollow interior received on the distal end of the injector body;
b) at least one finger flange (18) located at a proximal end of the injector body opposite the nozzle portion;
c) a plunger (16) adapted to reciprocate within the hollow interior of the injector body, the plunger having a plunger rod (26,26'); and
**characterized by**
d) a slidable gate (28,28') mounted on the injector body (14) adapted to engage the plunger rod and to prevent movement of the plunger rod.

2. The lens delivery system of claim 1, wherein the finger flange (18) has at least one slot (20), and the gate (28) has at least one tab (30), the tab sized and shaped to be received within the slot (20) so as to slidably retain the gate against the finger flange (18).

3. The lens delivery system of claim 1 or claim 2, wherein the gate (28) engages a notch (34) in the plunger rod (26).

4. The lens delivery system of claim 1, wherein the gate (28') further comprises a notch (55), and wherein the notch on the gate engages a rib (61) on the plunger rod (26').
